(19) Europäisches Patentamt
European Patent Office
Office européen des brevets

(11) **EP 4 038 158 B1**

(12) **EUROPEAN PATENT SPECIFICATION**

(45) Date of publication and mention
of the grant of the patent:
**24.12.2025 Bulletin 2025/52**

(21) Application number: **20793157.7**

(22) Date of filing: **02.10.2020**

(51) International Patent Classification (IPC):
**C09J 7/29** (2018.01)     **A61F 13/0246** (2024.01)
**A61M 25/02** (2006.01)

(52) Cooperative Patent Classification (CPC):
**C09J 7/29; A61F 13/0246;** A61M 2025/0266;
C09J 2301/124; C09J 2301/308; C09J 2301/312;
C09J 2400/22

(86) International application number:
**PCT/IB2020/059297**

(87) International publication number:
**WO 2021/064696 (08.04.2021 Gazette 2021/14)**

(54) **A FILM BACKING FOR RELEASABLE SECUREMENT**

FOLIENTRENNSCHICHT ZUR LÖSBAREN BEFESTIGUNG

SUPPORT DE FILM POUR FIXATION LIBÉRABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2019 US 201962910667 P**

(43) Date of publication of application:
**10.08.2022 Bulletin 2022/32**

(73) Proprietor: **Solventum Intellectual Properties
Company
Maplewood, MN 55144 (US)**

(72) Inventors:
• **LUDWIG, Bret W.**
**Saint Paul, Minnesota 55133-3427 (US)**
• **JOHNSON, Abbi G.**
**Saint Paul, Minnesota 55133-3427 (US)**
• **LENICZEK, Katelyn M.**
**Saint Paul, Minnesota 55133-3427 (US)**
• **STRAND, Lacey M.**
**Saint Paul, Minnesota 55133-3427 (US)**
• **EMSLANDER, Jeffrey O.**
**Saint Paul, Minnesota 55133-3427 (US)**
• **YOUNG, Jacob D.**
**Saint Paul, Minnesota 55133-3427 (US)**
• **KUDUVA RAMAN THANUMOORTHY,
Ramasubramani**
**Saint Paul, Minnesota 55133-3427 (US)**
• **SPIEWAK, Brian E.**
**Saint Paul, Minnesota 55133-3427 (US)**
• **NIES, Timothy J.**
**Saint Paul, Minnesota 55133-3427 (US)**
• **SIERACKI, James M.**
**Saint Paul, Minnesota 55133-3427 (US)**

(74) Representative: **Vossius & Partner
Patentanwälte Rechtsanwälte mbB
Siebertstraße 4
81675 München (DE)**

(56) References cited:
EP-A1- 1 397 098      WO-A1-2017/136432
US-A- 4 024 312       US-B1- 6 495 230

**Description**

**Technical Field**

The disclosure relates to a film backing for releasably securing a device to a surface, such as skin.

**Background**

Various medical devices are attached to a patient. For example, tubing, monitors, sensors, or catheters are secured to skin. To limit irritation, dislodgement, and potential exposure to infection, the medical devices should be securely attached to the patient. Adhesives and adhesive tapes are commonly used to secure devices to skin. Very strong adhesives can cause trauma to skin upon removal. A very gentle adhesive will remove from the skin easily but might not have sufficient strength to secure the medical device.

WO 2017/136432 A1 discloses a peelable adhesive article which is capable of adhering to a substrate wherein subsequent removal of the adhesive does not damage the substrate.

**Summary**

The subject-matter of the present invention is defined by the features of independent claim 1. Further preferred embodiments are defined in the dependent claims. The disclosed film backing has an extensible substrate with a securing adhesive that can secure to a surface and a tab to stretch the extensible substrate to remove the adhesive from the underlying surface. Stretching the extensible substrate will release the securing adhesive from the underlying surface easily and without trauma. Therefore, a strong adhesive can be used.

Stretch release tape are known for securing items to a surface, such as walls. For example, 3M™ Command™ tape is a stretch release tape with a foam substrate coated on both surfaces with a strong adhesive. One example of a low modulus material is described in PCT Publication WO2017/136432. Some stretch release materials have high elongation. Therefore, the extensible substrate must stretch significantly to release the adhesive from the underlying surface. In some application, there is simply insufficient space to allow for such stretching. Further, it is common that stretch release materials are elastic and the energy input to elongate the film can release when the adhesive detaches from the underlying substrate, which can cause snapping or catapulting of any overlying device.

When looking to secure a stretch release tape to an underlying substrate that is conformable, such as skin, the extensible substrate should have a low modulus to minimize the force required to stretch and pull the extensible substrate. The extensible substrate should have low elongation to minimize the distance needed to stretch and pull the extensible substrate. Further, it is desirable that a majority of the deformation of the extensible substrate be plastic deformation and that its elastic deformation be minimized. Plastic deformation of the extensible substrate reduces the energy released upon the removal of stress, since the energy input to the extensible substrate is consumed by the re-ordering the film morphology rather than stored as potential energy in elastic structures. Less stored energy improves safety during the removal of devices held in place with an extensible substrate. The combination of low modulus and large degree of plastic deformation makes one-handed stretch and removal of the extensible substrate from the surface possible, without the hazard of the extensible substrate and an overlying device, if included, from being snapped painfully into the removing hand.

To achieve plastic deformation, the disclosed extensible substrate has a core and at least first skin on one side of the core. In some embodiments, there is a first skin on a first side of the core and a second skin on the second, opposite, side of the core. Multilayer films designed for maximum strength often include an elastic layer forming the core sandwiched between two plastically-deforming layers forming the skin. To minimize the elasticity of the overall construction, the elastic content is minimized. The fraction of plastic material increases, and since plastics often have higher modulus values than elastics, the modulus of the film rises. It is possible to keep the modulus of the construction low enough to enable gentle removal from skin by making the total construction thinner. Typically, for embodiments with a single skin, the core thickness to skin thickness ratio may be less than 10:1. According to the invention the core thickness to skin thickness ratio is less than 2:1.

Because an advantage of a stretch release materials is that a stronger adhesive can be used for securing to the underlying substrate, a tab is included that has either reduced adhesion as compared to the stronger securing adhesive or is free-of a tacky adhesive. The tab allows the user to easily lift a portion of the extensible substrate and stretch the extensible substrate to remove the securing adhesive from the underlying substrate. In some embodiments, the disclosed film backing with an extensible substrate includes two, opposing tabs.

According to the invention, the film backing has a first major surface and a second major surface, opposite the first major surface. The film backing comprises extensible substrate comprising a core layer comprising an elastomeric material, a first skin layer adjacent to the core layer comprising a plastically deforming material, and a securing adhesive adjacent to the first major surface. The extensible substrate has a core thickness to first skin thickness ratio of less than 2 to 1, a plastic deformation of at least 50%, an elongation of less than 600%, and a Fn Modulus of less than 20 N per 2.54cm (1 inch) width at 50% elongation.

## Brief Description of Drawings

FIG. 1 is a side sectional view of one embodiment of a film backing;
FIG. 2 is a side sectional view of another embodiment of a film backing;
FIG. 3 is a side sectional view of another embodiment of a film backing;
FIG. 4 is a top perspective view of another embodiment of a film backing, similar to the embodiment of FIG. 2 or 3, with a device secured to the film backing;
FIG. 5 is side perspective view of the film backing of FIG. 4;
FIG. 6 is a top perspective view of another embodiment of a film backing with a device secured to the film backing;
FIG. 7 is a perspective view of an elongated strip of a film backing on a roll.

While the above-identified drawings and figures set forth embodiments of the invention, other embodiments are also contemplated, as noted in the discussion. In all cases, this disclosure presents the invention by way of representation and not limitation. The figures may not be drawn to scale.

## Detailed Description

Disclosed is a film backing comprising an extensible substrate with a securing adhesive adjacent to a first major surface of the extensible substrate. The extensible backing comprises a core layer comprising an elastomeric material and a first skin layer adjacent to the core layer comprising a plastically deforming material.

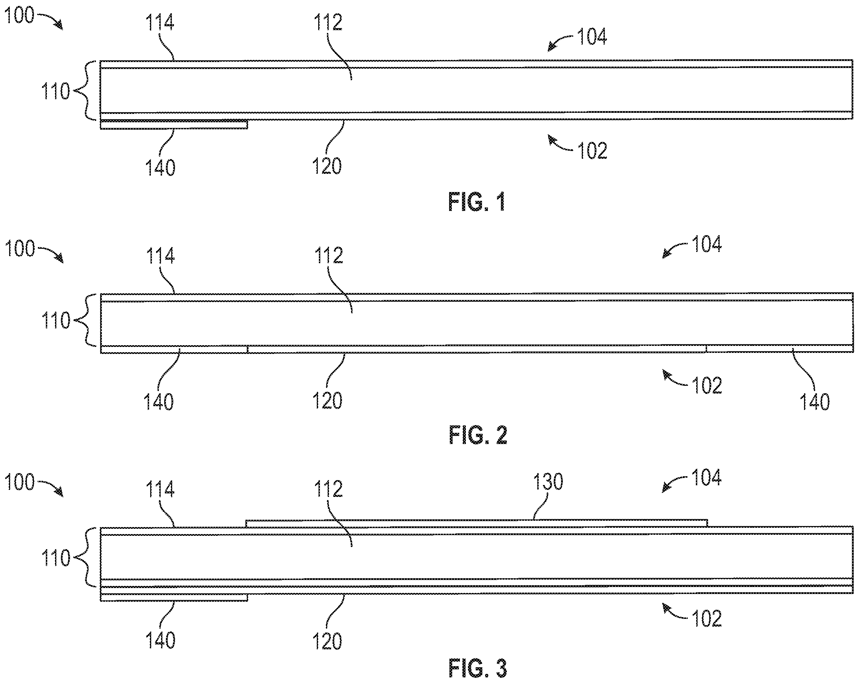

FIG. 1 is a side section of an embodiment of a film backing 100. The film backing 100 has a first major surface 102 and a second major surface 104, opposite the first major surface 102. The film backing 100 has an extensible substrate 110 comprising a core layer 112 comprising an elastomeric material and a first skin layer 114 adjacent to the core layer comprising a plastically deforming material. The film backing 100 has a securing adhesive 120 adjacent to the first major surface 102. The securing adhesive 120 secures the film substrate 110 to a surface 300.

FIG. 1 shows an optional tab 140. A tab 140 can be a portion of and edge of the film backing 100 with either reduced adhesion as compared to the securing adhesive 120 or is free-of a tacky adhesive. In this embodiment, tab 140 is form from a cover over the securing adhesive 120 to create a portion of the first major surface 102 that is free of a tacky adhesive. Therefore, a user can easily pull the extensible substrate 110 from the underlying surface to remove the extensible substrate 110 from the surface.

FIG. 2 is a side section of an embodiment of a film backing 100 similar to the embodiment in FIG. 1. In this embodiment, the securing adhesive 120 adjacent to the first major surface 102 and is on a portion of the first major surface 102. The securing adhesive 120 secures the film substrate 110 to a surface 300. In this embodiment, two tabs 140, on opposing sides of the film backing, are each at edge of the film backing 100 with either reduced adhesion as compared to the securing adhesive 120 or is free-of a tacky adhesive. In this embodiment, tab 140 is a second adhesive with reduced adhesion as compared to the securing adhesive 120. For example, a pressure sensitive adhesive that is repositionable, such as an acrylate or silicone adhesive can be at the tab 140 to create a portion of the first major surface 102 that a user can easily lift from the underlying surface and initiate stretching of the extensible substrate 110.

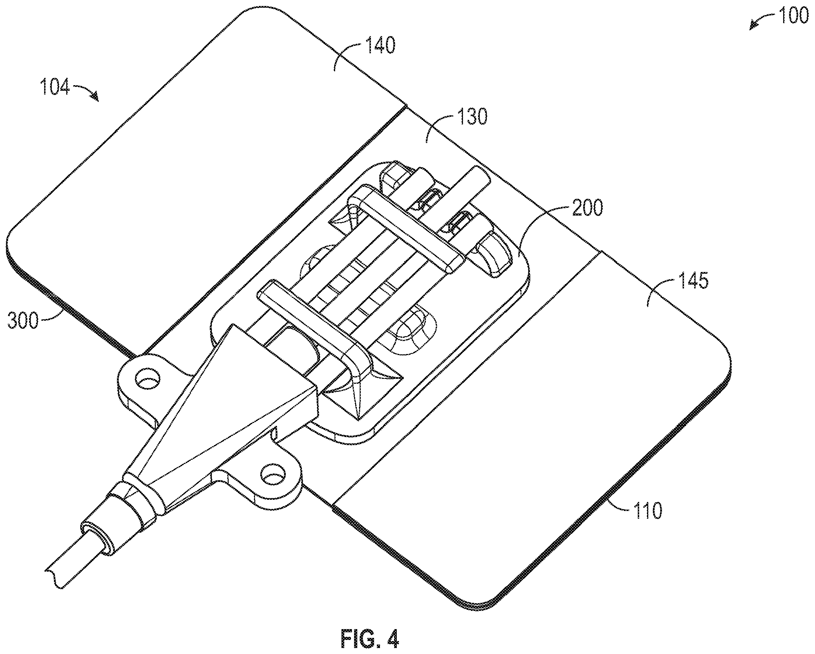

FIG. 3 is a side section of an embodiment of a film backing 100 similar to the embodiment in FIGS. 1 and 2. In this embodiment, a second adhesive 130 is adjacent to the second major surface 102. This second adhesive 130 can be used to secure to a device (shown in FIGS. 4-6). In this embodiment, the film backing 100 has a tab 140. In this embodiment the tab 140 includes a covering 144 over the securing adhesive 120.

To use the film backing 100, the securing adhesive 120 contacts a surface. To remove the film backing 100 from the surface, an edge of the film substrate 110 can be lifted and peeled from the surface. In embodiments with a tab 140, the tab 140 can be used to peel the film backing 100 from the surface 300. In embodiments with two opposing tabs 140, like shown in FIG. 2, both tabs 140 can be simultaneously pulled. This is advantageous as it limits or prevents lateral shifting of the central portion where the securing adhesive 120 is secured to the underlying surface.

The film backing 100 creates a stable, uniform surface for an overlying device to secure to. When the underlying substrate is skin, the texture, moisture, conformability and flexibility of the skin can vary significantly from one person to another. The disclosed film backing 100 forms a more uniform canvas for an overlying device to secure, such as shown in FIGS. 4-6. In the embodiment shown in FIG. 3, the film backing 100 includes a second adhesive to secure with an device, while the film backing 100 in FIGS. 1 and 2, the film backing 100 does not include this second adhesive and the device itself likely include an adhesive to secure to the film backing 100.

In some embodiments, the securing adhesive 120 is a strong adhesive and peeling the film backing 100 from the surface is challenging. Instead, the extensible substrate 110 is pulled with a shear force to longitudinally stretch the extensible substrate 110. This process is referred to as stretch release and results in elongation of the extensible substrate 110 and release of the securing adhesive 120 from the surface 300.

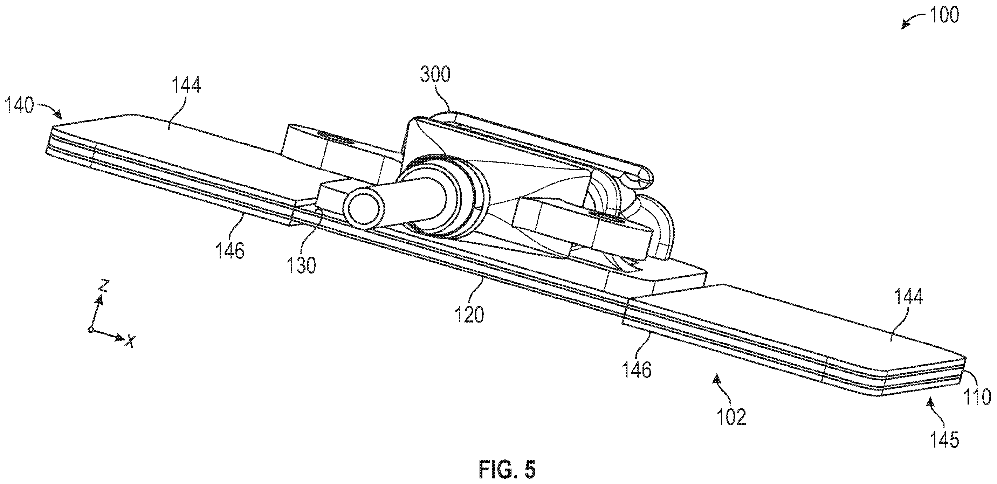

FIG. 4 is a top perspective view of another embodiment of a film backing 100, similar to the embodiment of FIG. 2 or 3, with a device 200 secured to the film backing 100. FIG. 5 is a side perspective view of the film backing of FIG. 4. Different from FIG. 3, in this embodiment the second adhesive 130 extends over the entire second major surface 104.

In this embodiment, covers 146 are included on the securing adhesive 120 at the first major surface 102 forming a first tab 140 and second tab 145. Instead of a cover, other materials or techniques could be used to reduce the adhesive properties of the securing adhesive 120. For example, films, coating, crosslinking, curing (radiation), can be used to reduce the adhesive properties of the second adhesive 130. Reducing the adhesive properties at the tab 140, 145 allow for the tab 140, 145 to be easily peeled from the underlying surface 300. Alternatively, it is understood that the securing adhesive 120 could be only provided at a portion of the first major surface 102. For example, the securing adhesive 120 could be provided at all of the first major surface 102, except for at the tab 140, 145. For example, the securing adhesive 120 could be included at the area underlying the device 200.

In this embodiment, covers 144 are included on the second adhesive 130 at the second major surface 104 forming a first tab 140 and second tab 145. Instead of a cover, other materials or techniques could be used to reduce the adhesive properties of the second adhesive 130. For example, films, coating, crosslinking, curing (radiation), can be used to reduce the adhesive properties of the second adhesive 130. Reducing the exposed adhesive on the second major surface 104 can prevent contaminants from contacting the second adhesive 130. In some embodiments, an overlying dressing (not shown), such as a 3M Tegaderm Dressing or a 3M Tegaderm IV Dressing, is included over the film backing 100 Alternatively, it is understood that the second adhesive 130 could be only provided at a portion of the second major surface 104. For example, the second adhesive 130 could be included at the area underlying the device 200.

In this embodiment, the extensible substrate 110 with the underlying securing adhesive 120 underlies the device 200 and extends beyond the perimeter of the device 200 forming a skirt around the device 200. As can be seen, the underlying securing adhesive 120 is vertically underlying the device 200 and the portion extending beyond the perimeter of the device 200. A skirt around the device 200 provides improved stability of the device 200 when attached to the surface 300.

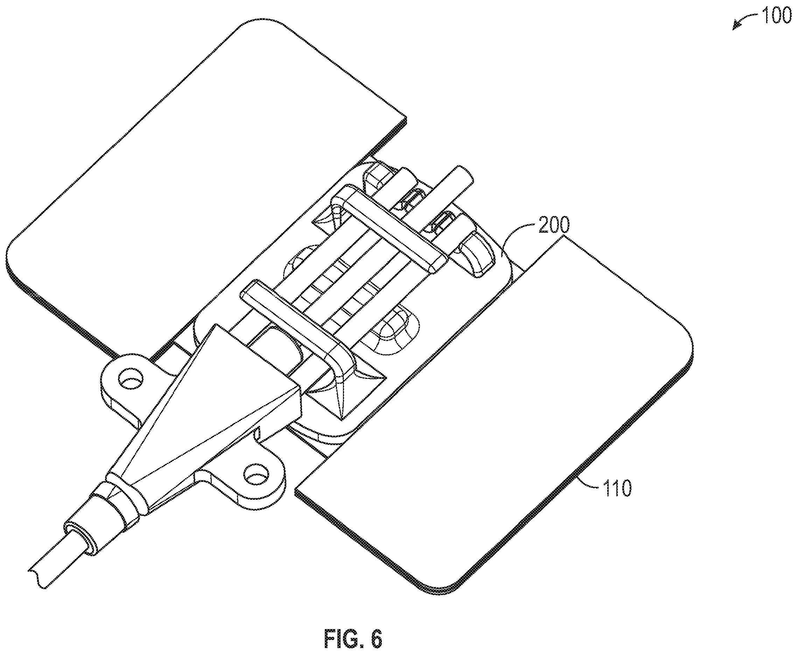

FIG. 6 is a perspective view of another embodiment of a film backing 100 with a device 200 secured to the film backing 100 similar to the film backing 100 shown in FIG. 4-5. Different from FIG. 4-5, in this embodiment the extensible substrate 110 extends beyond only a portion of the device 200 and directly under, but not beyond a portion of the device 200.

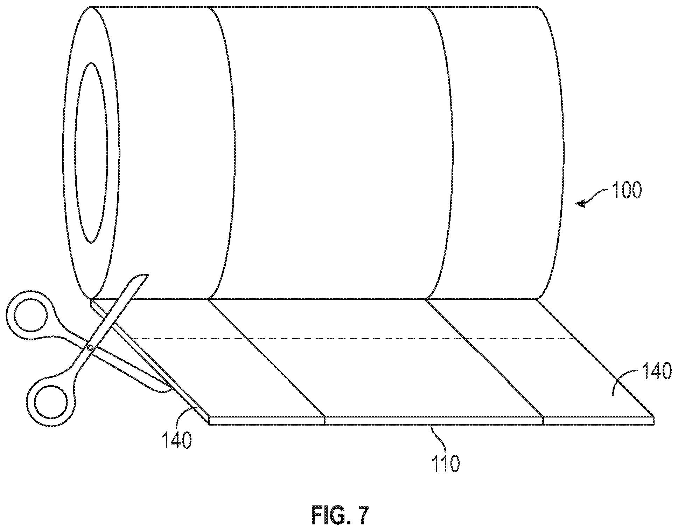

The film backing can be provided in a die cut strips or in an elongated strip that could be rolled. For example, FIG. 7 shows an embodiment of a roll of the film backing 100, which has a constructions similar to the embodiment of FIG. 2, where there are two opposing tabs 140 in either side of the extensible substrate 110. There could be preformed perforation areas to allow a user to remove a section or a user could cut a section. The elongated strip has a longitudinal length and a lateral length. In this embodiment, the tabs 140 provide for stretching of the extensible backing 110 in the direction of the lateral length.

**Materials**

The core comprises an elastomeric material. Elastomeric means that the material exhibits at least some ability to return at least in part to its original shape or size after forces causing the deformation are removed. The elastomeric material is selected from the group consisting of, an elastomeric polymer, SEBS, SEPS, SIS, SBS, polyurethane, ethyl vinylacetate (EVA), ethyl methyl acrylate (EMA) ultra low linear density polyethylene (ULLDPE), hydrogenated polypropylene, and combinations or blends thereof.

The skin comprises a plastically deforming material. Plastic deformation means that the material undergoes a permanent change in shape or size when subjected to a stress exceeding a particular value (the yield value). The plastically deforming material is selected from the group consisting of polypropylene, polyethylene, high density polyethylene (HDPE), low density polyethylene (LDPE), linear low density polyethylene (LLDPE), a polyurethane, EVA, EMA, an adhesive, and combinations or blends thereof.

The securing adhesive and second adhesive may be the same adhesive or different adhesive from one another. The adhesive could be a permanent adhesive for providing strong securement. The adhesive could be a repositionable adhesive, that provides securement but still allows for removing from peeling. The adhesive may be a pressure sensitive adhesive. A general description of useful pressure-sensitive adhesives may be found in the Encyclopedia of Polymer Science and Engineering, Vol. 13, Wiley-Interscience Publishers (New York, 1988). Additional description of useful pressure-sensitive adhesives may be found in the Encyclopedia of Polymer Science and Technology, Vol. I, Interscience Publishers (New York, 1964). Any suitable composition, material or ingredient can be used in the pressure-sensitive adhesive. Exemplary pressure-sensitive adhesives utilize one or more thermoplastic elastomers, e.g., in combination with one or more tackifying resins.

To achieve plastic deformation, the extensible substrate 110 is a multilayer construction of the core with at least one skin secured to the core. Typically, for embodiments with a single skin, the extensible substrate 110 may have a core thickness to skin thickness ratio less than 10:1. According to the invention the extensible substrate 110 has a core thickness to skin thickness ratio less than 2:1.

The core layer 112 and one or more skin layers 114, 116 can be bonded to one another using any suitable mechanism including, for example, coextruding the core and the skin layer(s), co-molding, extrusion coating, joining through an adhesive composition, joining under pressure, joining under heat, and combinations thereof.

In some embodiments, the adhesive layer can include at least one of rubber, silicone, or acrylic based adhesives. In some embodiments, the adhesive can include tackified rubber adhesives, such as natural rubber; olefins; silicones, such as silicone polyureas; synthetic rubber adhesives such as polyisoprene, polybutadiene, and styrene-isoprene-styrene, styrene-ethylenebutylene-styrene and styrene-butadiene-styrene block copolymers, and other synthetic elastomers; and tackified or untackified acrylic adhesives such as copolymers of isooctylacrylate and acrylic acid, which can be polymerized by radiation, solution, suspension, or emulsion techniques; polyurethanes; silicone block copolymers; and combinations of the above. The adhesive can be, for example, any of the adhesives described in any of the following patent applications: PCT Patent Publication Nos. 2015/035556, 2015/035960, and US 2015/034104. In some embodiments, the adhesive includes a tackifier. Some exemplary tackifiers include at least one of polyterpene, terpene phenol, rosin esters, and/or rosin acids.

**Extensible Substrate Properties**

Modulus and Elongation

The extensible substrate 110 has a low Fn Modulus. The Fn Modulus is the force required to elongate the extensible substrate 110. If a user is removing the film backing 100 from a location that cannot be accessed by both hands, the back of the arm for example, the user will need to remove it by pulling from an edge portion of the film backing 100 or from a single tab 140, if included. As the film backing 100 is pulled, the extensible substrate 110 stretches and the securing adhesive 120 releases from the surface beginning at the edge portion of the film backing and then proceeding along the extensible substrate 110. However, the force needed to stretch the extensible substrate 110 is also applied to the surface. If the surface is skin and if that force is excessive, it can become uncomfortable or even painful until the extensible substrate 110 releases from the skin.

Even in situations in which both hands can be used to hold the device and pull a tab or to pull opposing tabs, a low modulus will still make stretching and removal of the extensible substrate 110 easy for those with below-average strength or limited grip strength.

The extensible substrate 110 has a low elongation to minimize the distance needed to stretch and pull the extensible substrate 110. A very high elongation means the extensible substrate 110 will have a high distance it stretches before releasing from the surface 300. Excessive stretching is an inconvenience in settings where space is constrained.

The Fn Modulus is the force (F) required to elongate the test specimen a specified percent (n). Fn Modulus is usually recorded for elongation of 10%, 50%, and 100% for comparison of films. Elongation is the maximum percent of strain reached by a test sample to the point of breakage.

Modulus and elongation were measured under ambient conditions (25° C and 50% relative humidity) by a method based upon PSTC-31, ASTM D882 and D3759 Test Methods. The test was performed on a constant rate of extension/tensile tester (Instron, Zwick or equivalent) equipped for either English or Metric units and clamp-type jaws. Samples were razor cut approximately 10 cm longer than the gauge length (gauge length was 25.4 mm) and 25.4 mm in width. Cross head speed was 2286 mm/min.

The extensible substrate 110 ideal for application on skin has a F(50%) Modulus less than 20 N per 25.4 mm width. In one embodiment, the extensible substrate 110 has a F(50%) Modulus less than 10 N per 25.4 mm width at 50% strain. In one embodiment, the extensible substrate 110 has a F(50%) Modulus less than 5 N per 25.4 mm width.

The extensible substrate 110 has an elongation less than 600%. In one embodiment, the extensible substrate 110 has an elongation less than 500%. In one embodiment, the extensible substrate 110 has an elongation less than 600%. In one embodiment, the extensible substrate 110 has an elongation of at least 100%.

Plastic Deformation

It is desirable that a majority of the deformation of the extensible substrate 110 is plastic deformation and that its elastic deformation is minimized. Elasticity is the ability of a deformed material body to return to its original shape and size when the forces causing the deformation are removed. In contrast, plasticity is the property of a solid body whereby it undergoes a permanent change in shape or size when subjected to a stress exceeding a particular value (the yield value). A highly elastic film may present a hazard during stretching to remove the film from a surface because as the film stretches, a significant amount of energy is stored in it. If the stretching film is not secured, upon the release of the film from the surface this energy will accelerate the film, and any attached device, toward the hand stretching the film, possibly resulting in injury or a broken device.

Plastic deformation is calculated using the original length of the extensible substrate 110 (Lo), the length the extensible substrate 110 reached under applied stress (Ls) and the length the extensible substrate 110 relaxed to after the stress was removed (Lr).

$$\% \text{ Plastic deformation} = (\text{Lr-Lo})/(\text{Ls-Lo}) \times 100$$

For example, a one-inch (2.54cm) (Lo) length of film stretched to five inches (12.7cm) (Ls), which relaxes to a length of four inches (10.16cm) (Lr) when the applied stress is removed, has a plastic deformation of 75%. ((4-1)/5-1) $\times$ 100).

Plastic deformation of the extensible substrate 110 reduces the energy released upon the removal of stress, since the energy input to extensible substrate 110 is consumed by the re-ordering the film morphology rather than stored as potential energy in elastic structures. Less stored energy improves safety during the removal of devices held in place with an extensible substrate 110. The combination of low modulus and large degree of plastic deformation makes one-handed stretch and removal of the extensible substrate 110 from the surface possible, without the hazard of the extensible substrate 110 and an overlying device, if included, from being snapped painfully into the removing hand.

The extensible substrate 110 has a plastic deformation of at least 50%. In one embodiment, the extensible substrate 110 has a plastic deformation of at least 60%. In one embodiment, extensible substrate 110 has a plastic deformation of at least 70%.

Although specific embodiments have been shown and described herein, it is understood that these embodiments are merely illustrative of the many possible specific arrangements that can be devised in application of the principles of the invention.

**Claims**

1. A film backing (100) having a first major surface (102) and a second major surface (104), opposite the first major surface (102), the film backing (100) comprising

   an extensible substrate (110) comprising:

   a core layer (112) comprising an elastomeric material selected from the group consisting of, an elastomeric polymer, SEBS, SEPS, SIS, SBS, polyurethane, ethyl vinylacetate (EVA), ethyl methyl acrylate (EMA) ultra low linear density polyethylene (ULLDPE), hydrogenated polypropylene, and combinations or blends thereof;
   a first skin layer (114) adjacent to the core layer (112) comprising a plastically deforming material selected from the group consisting of polypropylene, polyethylene, high density polyethylene (HDPE), low density polyethylene (LDPE), linear low density polyethylene (LLDPE), a polyurethane, EVA, EMA, an adhesive, and combinations or blends thereof;
   a securing adhesive (120) adjacent to the first major surface (102);

   wherein the extensible substrate (110) has a core thickness to first skin thickness ratio of less than 2 to 1,
   wherein a length of the extensible substrate (110) after being stretched and relaxed is least 50% of a length of the extensible substrate when stressed less an original length of the extensible substrate before stretching,
   wherein a maximum precent of strain reached by the extensible substrate (110) to a point of breakage is less than 600%, and
   wherein a force required to elongate the extensible substrate (110) is less than 20 N per 25.4 mm width at 50% elongation;
   wherein the strain and the force are measured as set forth in the description.

2. The film backing (100) of claim 1, further comprising a second skin layer (116), adjacent to the core layer (112) and opposite from the first skin layer (114) wherein the second skin layer comprises a plastically deforming material selected from the group consisting of polypropylene, polyethylene, high density polyethylene (HDPE), low density polyethylene (LDPE), linear low density polyethylene (LLDPE), a polyurethane, EVA, EMA, an adhesive, and combinations or blends thereof.

3. The film backing (100) of any one of the preceding claims, further comprising a second adhesive (130) adjacent to the second major surface (104).

4. The film backing (100) of any one of the preceding claims, wherein the maximum percent of strain reached by the extensible substrate (110) to the point of breakage is at least 100% and less than 500%.

5. The film backing (100) of any one of the preceding claims, wherein the force required to elongate the extensible substrate (110) is less than 5 N per 25.4 mm width at 50% elongation.

6. The film backing (100) of any one of the preceding claims, further comprising a first tab (140) at a perimeter portion of the extensible substrate (110).

7. The film backing (100) of any one of the preceding claims further comprising a second tab (145).

8. The film backing (100) of claim 6 or 7, wherein at least one of the first major surface (102) or the second major surface (104) of the extensible substrate (110) at the first tab (140) and the second tab (145) are free of tacky adhesive.

9. The film backing (100) of any one of claims 6 to 8, wherein at the first major surface (102) of the extensible substrate (110) at the first tab (140) is a tab adhesive, different from the securing adhesive.

10. The film backing (100) of any one of claims 6 to 9, wherein at the second major surface (104) of the extensible substrate (110) at the first tab (140) is a tab adhesive, different from the securing adhesive.

11. The film backing (100) of any one of claims 3 to 10, wherein the securing adhesive (120) and second adhesive (130) are arranged in at least partially vertical overlapping relationship to one another.

12. The film backing (100) of any one of claims 3 to 11, further comprising a device secured to the second adhesive (130).

13. The film backing (100) of claim 12, wherein the extensible substrate (110) extends beyond a perimeter of the device.

**Patentansprüche**

1. Ein Filmträger (100), der eine erste Hauptoberfläche (102) und eine zweite Hauptoberfläche (104) gegenüber der ersten Hauptoberfläche (102) aufweist, der Filmträger (100) aufweisend
   ein dehnbares Substrat (110), aufweisend:

   eine Kernschicht (112), aufweisend ein Elastomermaterial, das aus der Gruppe ausgewählt ist, bestehend aus einem Elastomerpolymer, SEBS, SEPS, SIS, SBS, Polyurethan, Ethylvinylacetat (EVA), Ethylmethylacrylat (EMA), linearem Polyethylen ultraniedriger Dichte (ULLDPE), hydriertem Polypropylen und Kombinationen oder Mischungen davon;
   eine erste Hautschicht (114) angrenzend an die Kernschicht (112), aufweisend ein plastisch verformendes Material, das aus der Gruppe ausgewählt ist, bestehend aus Polypropylen, Polyethylen, Polyethylen hoher Dichte (HDPE), Polyethylen niedriger Dichte (LDPE), linearem Polyethylen niedriger Dichte (LLDPE), einem Polyurethan, EVA, EMA, einem Klebstoff und Kombinationen oder Mischungen davon;
   einen Befestigungsklebstoff (120) angrenzend an die erste Hauptoberfläche (102);
   wobei das dehnbare Substrat (110) ein Verhältnis von Kerndicke zu Dicke erster Haut von weniger als 2 zu 1 aufweist,
   wobei eine Länge des dehnbaren Substrats (110) nach einem Strecken und Entspannen mindestens 50 % einer Länge des dehnbaren Substrats, wenn gespannt abzüglich einer ursprünglichen Länge des dehnbaren Substrats vor dem Strecken beträgt,
   wobei ein maximaler Prozentsatz von Beanspruchung, der durch das dehnbare Substrat (110) bis zu einem Bruchpunkt erreicht wird, weniger als 600 % beträgt, und
   wobei eine Kraft, die benötigt wird, um das dehnbare Substrat (110) zu verlängern, weniger als 20 N pro 25,4 mm Breite bei 50 % Verlängerung beträgt; wobei die Beanspruchung und die Kraft wie in der Beschreibung dargelegt gemessen werden.

2. Der Filmträger (100) nach Anspruch 1, ferner aufweisend eine zweite Hautschicht (116) angrenzend an die Kernschicht (112) und gegenüber von der ersten Hautschicht (114), wobei die zweite Hautschicht ein plastisch verformendes Material aufweist, das aus der Gruppe ausgewählt ist, bestehend aus Polypropylen, Polyethylen, Polyethylen hoher Dichte (HDPE), Polyethylen niedriger Dichte (LDPE), linearem Polyethylen niedriger Dichte (LLDPE), einem Polyurethan, EVA, EMA, einem Klebstoff und Kombinationen oder Mischungen davon.

3. Der Filmträger (100) nach einem der vorstehenden Ansprüche, ferner aufweisend einen zweiten Klebstoff (130) angrenzend an die zweite Hauptoberfläche (104).

4. Der Filmträger (100) nach einem der vorstehenden Ansprüche, wobei der maximale Prozentsatz von Beanspruchung, der durch das dehnbare Substrat (110) bis zu dem Bruchpunkt erreicht wird, mindestens 100 % und weniger als 500 % beträgt.

5. Der Filmträger (100) nach einem der vorstehenden Ansprüche, wobei die Kraft, die benötigt wird, um das dehnbare Substrat (110) zu verlängern, weniger als 5 N pro 25,4 mm Breite bei 50 % Verlängerung beträgt.

6. Der Filmträger (100) nach einem der vorstehenden Ansprüche, ferner aufweisend eine erste Lasche (140) an einem Umfangsabschnitt des dehnbaren Substrats (110).

7. Der Filmträger (100) nach einem der vorstehenden Ansprüche, ferner aufweisend eine zweite Lasche (145).

8. Der Filmträger (100) nach Anspruch 6 oder 7, wobei mindestens eine von der ersten Hauptoberfläche (102) oder der zweiten Hauptoberfläche (104) des dehnbaren Substrats (110) an der ersten Lasche (140) und der zweiten Lasche (145) frei von klebrigem Klebstoff sind.

9. Der Filmträger (100) nach einem der Ansprüche 6 bis 8, wobei an der ersten Hauptoberfläche (102) des dehnbaren Substrats (110) an der ersten Lasche (140) ein Laschenklebstoff ist, der sich von dem Befestigungsklebstoff unterscheidet.

10. Der Filmträger (100) nach einem der Ansprüche 6 bis 9, wobei an der zweiten Hauptoberfläche (104) des dehnbaren Substrats (110) an der ersten Lasche (140) ein Laschenklebstoff ist, der sich von dem Befestigungsklebstoff unterscheidet.

11. Der Filmträger (100) nach einem der Ansprüche 3 bis 10, wobei der Befestigungsklebstoff (120) und der zweite Klebstoff (130) in einer mindestens teilweise vertikalen überlappenden Beziehung zueinander angeordnet sind.

12. Der Filmträger (100) nach einem der Ansprüche 3 bis 11, ferner aufweisend eine Vorrichtung, die an den zweiten Klebstoff (130) befestigt ist.

13. Der Filmträger (100) nach Anspruch 12, wobei sich das dehnbare Substrat (110) über einen Umfang der Vorrichtung hinaus erstreckt.

**Revendications**

1. Support de film (100) ayant une première surface principale (102) et une seconde surface principale (104), opposée à la première surface principale (102), le support de film (100) comprenant un substrat extensible (110) comprenant :

une couche centrale (112) comprenant un matériau élastomère choisi dans le groupe constitué d'un polymère élastomère, SEBS, SEPS, SIS, SBS, polyuréthane, éthylvinylacétate (EVA), éthylméthylacrylate (EMA), polyéthylène linéaire ultra basse densité (ULLDPE), polypropylène hydrogéné, et des combinaisons ou mélanges de ceux-ci ;
une première couche de peau (114) adjacente à la couche centrale (112) comprenant un matériau à déformation plastique choisi dans le groupe constitué de polypropylène, polyéthylène, polyéthylène haute densité (PEHD), polyéthylène basse densité (PEBD), polyéthylène basse densité linéaire (PEBDL), un polyuréthane, EVA, EMA, un adhésif, et des combinaisons ou mélanges de ceux-ci ;

un adhésif de fixation (120) adjacent à la première surface principale (102) ;

dans lequel le substrat extensible (110) a un rapport entre l'épaisseur centrale et l'épaisseur de première peau inférieur à 2 pour 1,

dans lequel une longueur du substrat extensible (110), après avoir été étiré et détendu, est d'au moins 50 % d'une longueur du substrat extensible lorsqu'il est soumis à une contrainte moins une longueur originale du substrat extensible avant l'étirement,

dans lequel un pourcentage maximal de déformation atteint par le substrat extensible (110) jusqu'à un point de rupture est inférieur à 600 %, et

dans lequel une force nécessaire pour allonger le substrat extensible (110) est inférieure à 20 N pour 25,4 mm de largeur à 50 % d'allongement ; dans lequel la déformation et la force sont mesurées comme indiqué dans la description.

2. Support de film (100) selon la revendication 1, comprenant en outre une seconde couche de peau (116), adjacente à la couche centrale (112) et opposée à la première couche de peau (114), dans lequel la seconde couche de peau comprend un matériau à déformation plastique choisi dans le groupe constitué de polypropylène, polyéthylène, polyéthylène haute densité (PEHD), polyéthylène basse densité (PEBD), polyéthylène basse densité linéaire (PEBDL), un polyuréthane, EVA, EMA, un adhésif, et des combinaisons ou mélanges de ceux-ci.

3. Support de film (100) selon l'une quelconque des revendications précédentes, comprenant en outre un second adhésif (130) adjacent à la seconde surface principale (104).

4. Support de film (100) selon l'une quelconque des revendications précédentes, dans lequel le pourcentage maximal de déformation atteint par le substrat extensible (110) au point de rupture est au moins de 100 % et inférieur à 500 %.

5. Support de film (100) selon l'une quelconque des revendications précédentes, dans lequel la force nécessaire pour allonger le substrat extensible (110) est inférieure à 5 N pour 25,4 mm de largeur à 50 % d'allongement.

6. Support de film (100) selon l'une quelconque des revendications précédentes, comprenant en outre une première languette (140) au niveau d'une partie de périmètre du substrat extensible (110).

7. Support de film (100) selon l'une quelconque des revendications précédentes, comprenant en outre une seconde languette (145).

8. Support de film (100) selon la revendication 6 ou 7, dans lequel au moins l'une de la première surface principale (102) ou de la seconde surface principale (104) du substrat extensible (110) au niveau de la première languette (140) et de la seconde languette (145) est exempte d'adhésif collant.

9. Support de film (100) selon l'une quelconque des revendications 6 à 8, dans lequel, au niveau de la première surface principale (102) du substrat extensible (110), au niveau de la première languette (140), se trouve un adhésif de languette, différent de l'adhésif de fixation.

10. Support de film (100) selon l'une quelconque des revendications 6 à 9, dans lequel, au niveau de la seconde surface principale (104) du substrat extensible (110), au niveau de la première languette (140), se trouve un adhésif de languette, différent de l'adhésif de fixation.

11. Support de film (100) selon l'une quelconque des revendications 3 à 10, dans lequel l'adhésif de fixation (120) et le second adhésif (130) sont disposés en relation de chevauchement au moins partiellement verticale l'un par rapport à l'autre.

12. Support de film (100) selon l'une quelconque des revendications 3 à 11, comprenant en outre un dispositif fixé au second adhésif (130).

13. Support de film (100) selon la revendication 12, dans lequel le substrat extensible (110) s'étend au-delà d'un périmètre du dispositif.

FIG. 1

FIG. 2

FIG. 3

10

FIG. 4

EP 4 038 158 B1

FIG. 5

EP 4 038 158 B1

FIG. 6

EP 4 038 158 B1

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017136432 A1
- WO 2017136432 A
- US 2015035556 W
- US 2015035960 W
- US 2015034104 A

**Non-patent literature cited in the description**

- Encyclopedia of Polymer Science and Engineering. Wiley-Interscience Publishers, 1988, vol. 13
- Encyclopedia of Polymer Science and Technology. Interscience Publishers, 1964, vol. I